# EUROPEAN PATENT APPLICATION

(11) **EP 4 656 093 A1**
(43) Date of publication of application: **03.12.2025**
(21) Application number: 24178361.2
(22) Date of filing: 28.05.2024
(51) Int. Cl.: A45D 27/46, A61L 2/10, B26B 19/38

(54) **SANITIZING DEVICE FOR DISINFECTING A SHAVING UNIT OF A SHAVING DEVICE BY MEANS OF DISINFECTING LIGHT**

(71) Applicant: Koninklijke Philips N.V., 5656 AG Eindhoven (NL)
(72) Inventor: GOKHARU, Rajkumar Hiralal, 5656 AG Eindhoven (NL); OTO, Fatma Aytuna, 5656 AG Eindhoven (NL); PEETERS, Ceciel Wilhelmina Maria, 5656AG Eindhoven (NL); HALBERTSMA, Eelke Tjomme, 5656 AG Eindhoven (NL); VAN PEER WARBURTON, Mariska, 5656 AG Eindhoven (NL)
(74) Representative: Philips Intellectual Property & Standards

(57) **Abstract**

The invention relates to a sanitizing device (1) for disinfecting a shaving unit (7) of an electric shaving device (3) by means of disinfecting light. The sanitizing device has a base member (31) having a first cavity (43) for receiving a supporting member (15) of the shaving unit when the shaving unit is decoupled from a main body (5) of the shaving device. The sanitizing device further has a cover member (33) having a second cavity (71) for receiving, when the cover member is in a closing position relative to the base member, a hair-cutting unit (9a, 9b, 9c) of the shaving unit arranged on the supporting member. A light source (81a, 81b, 81c) is arranged in the cover member for irradiating the hair-cutting unit with the disinfecting light when the cover member is in the closing position wherein the first cavity and the second cavity fully enclose the shaving unit.

## Description

### FIELD OF THE INVENTION

The invention relates to a sanitizing device configured to disinfect a shaving unit of an electric shaving device by means of disinfecting light.

The invention also relates to a shaving system comprising an electric shaving device and a sanitizing device configured to disinfect a shaving unit of the shaving device by means of disinfecting light.

### BACKGROUND OF THE INVENTION

Electric shaving devices are widely known. Electric shaving devices usually have a shaving unit which is arranged on a main body or handle of the shaving device. The main body may accommodate an electric motor, configured to drive the shaving unit, and a battery to power the motor. The shaving unit may comprise one or more hair-cutting units, which may have an external cutting member with hair entry openings and an internal cutting member which is covered by and moveable relative to the external cutting member. In known examples the internal cutting member has a plurality of cutting blades and is linearly reciprocating within a foil-type external cutting member. In other known examples, the hair-cutting unit is of a rotary type, wherein the internal cutting member has an annular array of cutting elements and is rotatable within an external cutting member having an annular shaving track with hair-entry openings. In a well-known example the shaving unit has three rotary-type hair-cutting units arranged triangularly on a supporting member of the shaving unit. The shaving unit may be fixedly mounted on the main body or may be releasably coupled to the main body. In the latter case, the main body may be provided with a first coupling member and the supporting member of the shaving unit may be provided with a second coupling member configured to be releasably couplable to the first coupling member for coupling and decoupling of the shaving unit to and from the main body. The second coupling member may be arranged at a first main side of the supporting member, which faces the main body when the shaving unit is coupled to the main body, and the one or more hair-cutting units may be arranged at a second main side of the supporting member opposite to the first main side.

After one or more shaving sessions with such an electric shaving device, the shaving unit of the shaving device may need to be cleaned and/or disinfected. In this respect, it is known to use an electric shaving device with a disinfecting device that is configured to generate ultraviolet, UV, disinfecting light, or another type of disinfecting light, to disinfect the shaving unit. UV light is known for its strong disinfecting properties and is effective in inactivating microorganisms by damaging their DNA and RNA. Disinfection of the shaving unit will reduce the risk of skin infection and inflammation by microorganisms on the shaving unit when in contact with the skin during a next shaving session.

US 2005/0189907 A1 discloses a sanitizing device configured to disinfect the shaving unit of an electric shaving device by means of disinfecting light. The known sanitizing device comprises a base member having a longitudinal receiving groove formed on the upper surface of the base member. The receiving groove is configured to stably receive and mount an electric shaving device, including its main body and its shaving unit, in a position which is inclined relative to a horizontal position. When the shaving device is received by the receiving groove, a battery terminal, provided on an end portion of the main body of the shaving device remote from the shaving unit, is in electrical contact with a charging terminal provided on the base member of the sanitizing device. This allows a battery in the main body of the shaving device to be charged when the shaving device is placed in the sanitizing device. When the shaving device is received by the receiving groove, the shaving unit of the shaving device is in a position adjacent to a UV lamp, which is also arranged on the upper surface of the base member of the sanitizing device. This allows the shaving unit to be disinfected by the UV light generated by the UV lamp. The sanitizing device further comprises a cover which is hinged onto the base member. By closing the cover, the receiving groove accommodating the shaving device is fully covered. An on/off switch to active the UV lamp is also arranged on the upper surface of the base member and is turned on by contact with the cover when the cover is pivoted into the closed position. Although the known sanitizing device is able to provide both an electrical charging function and a disinfecting function, a disadvantage of the known sanitizing device is that it has a rather bulky and heavy structure. As a result, it is not convenient for the user to use this known sanitizing device, together with the shaving device, as a so-called "on-the-go" product suitable for taking in the luggage during travelling.

### SUMMARY OF THE INVENTION

It is an object of the present invention to provide a sanitizing device, configured to disinfect a shaving unit of an electric shaving device by means of disinfecting light, which has a compact and light structure and, at the same time, is safe to use by preventing the disinfecting light from injuring the user's eyes.

To achieve the above-mentioned object, the present invention provides a sanitizing device configured to disinfect a shaving unit of an electric shaving device by means of disinfecting light, wherein the shaving device comprises a main body provided with a first coupling member and the shaving unit comprises:
- a supporting member provided with a second coupling member configured to be releasably couplable to the first coupling member for coupling and decoupling of the shaving unit to and from the main body, wherein the second coupling member is arranged at a first main side of the supporting member which faces the main body when the shaving unit is coupled to the main body; and
- at least one hair-cutting unit arranged at a second main side of the supporting member opposite to the first main side;
wherein the sanitizing device comprises:
- a base member comprising a first cavity configured to at least partially receive the supporting member of the shaving unit in a condition of the shaving unit decoupled from the main body, said first cavity having a first main inner surface configured to support the first main side of the supporting member in a predefined sanitizing position of the shaving unit with respect to the base member;
- a cover member having a closing position and an opening position relative to the base member, and further having a second cavity configured to receive, when the cover member is in the closing position and the shaving unit is in the predefined sanitizing position, the at least one hair-cutting unit of the shaving unit, said second cavity having a second main inner surface; and
- at least one light source configured to generate the disinfecting light and arranged in the cover member to irradiate, from a light emitting area at the second main inner surface, the at least one hair-cutting unit with the disinfecting light when the cover member is in the closing position and the shaving unit is in the predefined sanitizing position;
wherein the first cavity and the second cavity are configured to fully enclose the shaving unit in its condition decoupled from the main body in the closing position of the cover member when the shaving unit is in the sanitizing position.

The sanitizing device according to the invention is configured to only receive the shaving unit of the shaving device, i.e., when the shaving unit is decoupled from the main body of the shaving device. The first cavity, which is provided in the base member of the sanitizing device, defines a predefined sanitizing position of the shaving unit with respect to the base member, wherein the first main side of the supporting member of the shaving unit is supported by the first main inner surface of the first cavity. The second cavity, which is provided in the cover member of the sanitizing device, is configured to receive the at least one hair-cutting unit of the shaving unit, i.e., when the shaving unit is in the predefined sanitizing position and the cover member is in the closing position. In said closing position, the disinfecting light generated by the at least one light source is applied to the at least one hair-cutting unit from the light emitting area at the second main inner surface of the second cavity. By supporting the shaving unit at the first main side of the supporting member of the shaving unit by means of the first main inner surface of the first cavity, and by providing the light emitting area at the second main inner surface of the second cavity to enable the irradiation of the at least one hair-cutting unit which is arranged at the second main side of the supporting member of the shaving unit, the shape and dimensions of the first cavity and the second cavity can be adapted to the shape and dimensions of the shaving unit, so that the dimensions of the first cavity and the second cavity required to accommodate the shaving unit can be minimized, thereby enabling a compact and light structure of the sanitizing device. Because in the closing position of the cover member the first cavity and the second cavity fully enclose the shaving unit, the disinfecting light irradiated from the light emitting area at the second main inner surface of the second cavity is prevented from escaping from the first cavity and the second cavity towards the environment of the sanitizing device and, thereby, prevented from injuring the user's eyes.

In an embodiment of the sanitizing device according to the invention, wherein the second coupling member of the shaving unit comprises a shaft-like coupling element which protrudes from the first main side of the supporting member of the shaving unit, the first cavity of the base member comprises a recess which is provided at the first main inner surface of the first cavity and configured to receive the shaft-like coupling element when the shaving unit is in the sanitizing position. Shaving units comprising a shaft-like coupling element protruding from the first main side of the supporting member are well-known. Such shaving units usually have two or more hair-cutting units which are driven by a single drive shaft arranged in the shaft-like coupling element. The shaft-like coupling element is configured to be received by a coupling recess provided in the main body of the shaving device. In this embodiment, the recess provided at the first main inner surface of the first cavity of the base member is configured to receive the shaft-like coupling element of the shaving unit. This enables direct contact of the first main side of the supporting member with the first main inner surface of the first cavity in the predefined sanitizing position of the shaving unit, despite the presence of the protruding shaft-like coupling element at the first main side of the supporting member, so that the dimensions of the first cavity required to accommodate the shaving unit can be limited as much as possible despite the presence of the shaft-like coupling element on the shaving unit. In addition, the recess may assist the user placing the shaving unit in the predefined sanitizing position within the base member of the sanitizing device. In a particular example of this embodiment of the sanitizing device according to the invention, wherein the shaving unit comprises three rotary-type hair-cutting units arranged triangularly at the second main side of the supporting member, and wherein a central axis of the shaft-like coupling element extends centrally between the three hair-cutting units, the recess of the first cavity of the base member is arranged centrally at the first main inner surface of the first cavity.

In a further embodiment of the sanitizing device according to the invention, the base member has a drainage conduit configured to connect a first drainage opening, which is provided in the recess of the first cavity, with a second drainage opening which is provided in a bottom surface of the base member. For an effective disinfecting process by means of the disinfecting light, the shaving unit and in particular the hair-cutting units thereof need to be first cleaned by means of water or a cleaning fluid in order to remove any debris from the shaving unit and the hair-cutting units. By providing the drainage conduit, the user does not need to dry or fully dry the shaving unit before placing the shaving unit in the sanitizing device. Water or cleaning fluid present on the shaving unit when placing the shaving unit in the sanitizing device may drip from the shaving unit and flow into the recess of the first cavity and may be drained via the first drainage opening, the drainage conduit and the second drainage opening out of the base member. Allowing the water or cleaning fluid to be drained from the sanitizing device mitigates the risk of an ineffective disinfecting process by the disinfecting light as a result of the presence of any water or cleaning fluid on the shaving unit. Preferably, the first drainage opening and the second drainage opening are not arranged in line to reduce as much as possible the transmission of any disinfecting light via the drainage conduit to the second drainage opening.

In an embodiment of the sanitizing device according to the invention, the base member has a bottom surface and a central axis extending perpendicularly to the bottom surface, and the first main inner surface of the first cavity, seen in a cross section which includes the central axis, has a main direction of extension which is inclined relative to an imaginary plane extending perpendicularly to the central axis of the base member. In this embodiment, when the bottom surface of the base member is placed on a horizontal external supporting surface, e.g., of a table, the first main inner surface of the first cavity of the base member is inclined relative to a horizontal imaginary plane, seen in said cross section. As a result, in the predefined sanitizing position the shaving unit is also inclined relative to the horizontal imaginary plane. This improves the visibility of the first cavity of the base member for the user when placing the shaving unit in the base member. The inclined main orientation of the first main inner surface of the first cavity also enables any water or cleaning fluid dripping from the shaving unit to flow towards a single lowest area of the first cavity. In a particular example of this embodiment of the sanitizing device according to the invention, wherein the shaving unit comprises three rotary-type hair-cutting units arranged triangularly at the second main side of the supporting member, the first main inner surface of the first cavity is configured such that, in the predefined sanitizing position of the shaving unit, a first one and a second one of the three hair-cutting units of the shaving unit are arranged at a first distance from the bottom surface of the base member, and a third one of the three hair-cutting units of the shaving unit is arranged at a second distance from the bottom surface which is smaller than the first distance. In this particular example of this embodiment of the sanitizing device according to the invention, the base member may have a drainage conduit configured to connect a first drainage opening, which is provided in the first main inner surface of the first cavity, with a second drainage opening which is provided in a bottom surface of the base member, wherein the first drainage opening is arranged in a position which is adjacent to the third one of the three hair-cutting units of the shaving unit when the shaving unit is in the sanitizing position. Preferably, when the sanitizing device is placed on a horizontal external supporting surface, the first drainage opening is present at a lowest position of the inclined first main inner surface of the first cavity. Preferably, the first drainage opening and the second drainage opening are not arranged in line to reduce as much as possible the transmission of any disinfecting light via the drainage conduit to the second drainage opening.

In an embodiment of the sanitizing device according to the invention, wherein the shaving unit comprises three rotary-type hair-cutting units arranged triangularly at the second main side of the supporting member, the first main inner surface of the first cavity is:
- surrounded by mutually opposite first and second side surfaces of the first cavity and by mutually opposite third and fourth side surfaces of the first cavity which each extend between the first and second side surfaces;
- configured such that, in the predefined sanitizing position of the shaving unit, a first one and a second one of the three hair-cutting units of the shaving unit are arranged adjacent to the first side surface on an imaginary line extending between the third and fourth side surfaces, and a third one of the three hair-cutting units of the shaving unit is arranged in a central position relative to and adjacent to the second side surface.

In this embodiment the first cavity may have a generally square or quadrangular shape, or a rounded generally square or quadrangular shape defined by said first, second, third and fourth side surfaces. By defining the sanitizing position of the shaving unit such that, in the sanitizing position, the first one and second one of the three hair-cutting units of the shaving unit are arranged adjacent to the first side surface of the first cavity and the third one of the three hair-cutting units of the shaving unit is arranged in a central position relative to and adjacent to the second side surface of the first cavity opposite to the first side surface, two open spaces are available, respectively, between the third side surface of the first cavity and the third one of the three hair-cutting units and between the fourth side surface of the first cavity and the third one of the three hair-cutting units when the shaving unit is in the sanitizing position within the first cavity. Said two open spaces are accessible for the user's fingers when placing the shaving unit into the first cavity and when removing the shaving unit from of the first cavity, thus facilitating the placement and removal of the shaving unit by the user into and from the sanitizing device. In this embodiment, apart from these two open spaces, the first, second, third and fourth side surfaces of the first cavity may closely surround the shaving unit in the sanitizing position, so that the dimensions of the first cavity can be minimized and, thereby, the sanitizing device can be as compact as possible. In this embodiment of the sanitizing device according to the invention, the first and second side surfaces of the first cavity may each be connected to the third and fourth side surfaces of the first cavity by a respective one of four curved side-surface portions of the first cavity. In particular, the curved side-surface portions which connect the first side surface with the third side surface and the fourth side surface may be shaped to closely surround the first one and the second one of the three hair-cutting units of the shaving unit in the predefined sanitizing position of the shaving unit.

It is to be noted that the second cavity, which is provided in the cover member, may generally have a circumferential edge portion having a shape which is similar to the shape of a circumferential edge portion of the first cavity provided in the base member such that, in the closing position of the cover member, the circumferential edge portions of the first cavity and the second cavity may be in mutual contact over the full circumference of the base member and the cover member.

In the sanitizing device in accordance with the invention, preferably the cover member is connected to the base member by means of a hinge structure, and the cover member is pivotable relative to the base member between the opening position and the closing position about a hinge axis defined by the hinge structure. However, the invention also covers embodiments wherein, to realize the opening position of the cover member relative to the base member, the cover member may be moveably coupled to the base member in a different manner or may be completely removed from the base member. Preferably, the cover member comprises a detector, which is configured and arranged to detect whether the cover member is in the closing position and to disable the at least one light source from generating the disinfecting light when the cover member is in the opening position. The detector thus prevents the activation of the light source when the cover member is in the opening position relative to the base member and, thereby, prevents injury to the user's eyes by the disinfecting light when the cover member is in the opening position.

In an embodiment of the sanitizing device according to the invention, wherein the shaving unit comprises three rotary-type hair-cutting units arranged triangularly at the second main side of the supporting member, the sanitizing device comprises three light sources arranged triangularly in the cover member to each irradiate, from a respective one of three triangularly arranged light emitting areas at the second main inner surface of the second cavity, a respective one of the three hair-cutting units with the disinfecting light when the cover member is in the closing position and the shaving unit is in the predefined sanitizing position. In this embodiment, the three light emitting areas may be arranged in positions at the second main inner surface of the second cavity corresponding to the positions of the three hair-cutting units at the second main side of the supporting member of the shaving unit, i.e., in the sanitizing position of the shaving unit within the sanitizing device. Since in this embodiment the sanitizing device has a separate light source and light emitting area associated with each individual hair-cutting unit of the shaving unit, the light sources and light emitting areas may each be arranged in positions relatively close to the associated hair-cutting unit for an effective disinfection of the hair-cutting unit. The arrangement of the light sources and light emitting areas in positions close to the shaving unit also reduces the dimensions of the sanitizing device.

In an embodiment of the sanitizing device according to the invention, the at least one light source is arranged on a printed circuit board which is arranged in the cover member between the second main inner surface of the second cavity and a top surface of the cover member. This arrangement of the light source results in a compact structure of the sanitizing device. The light source may be an LED, e.g., a UV LED. In this embodiment of the sanitizing device according to the invention, the sanitizing device may comprise a user-operable on/off button arranged at the top surface of the cover member and electrically connected to the printed circuit board. This allows the arrangement of all main electrical components of the sanitizing device in the cover member, which further simplifies the structure of the sanitizing device.

The present invention further provides a shaving system comprising an electric shaving device and a sanitizing device according to any of the embodiments of the invention as described herein before configured to disinfect a shaving unit of the shaving device by means of disinfecting light, wherein the shaving device comprises a main body provided with a first coupling member, and wherein the shaving unit of the shaving device comprises:
- a supporting member provided with a second coupling member configured to be releasably couplable to the first coupling member for coupling and decoupling of the shaving unit to and from the main body, wherein the second coupling member is arranged at a first main side of the supporting member which faces the main body when the shaving unit is coupled to the main body; and
- at least one hair-cutting unit arranged at a second main side of the supporting member opposite to the first main side.

The above-described and other aspects of the invention will be apparent from and elucidated with reference to the following detailed description of embodiments of an electric hair-cutting device in accordance with the invention.

### BRIEF DESCRIPTION OF THE DRAWINGS

The invention will be explained in greater detail with reference to the figures, in which equal or similar features are indicated by the same reference numbers, and in which:
Fig. 1 shows an embodiment of a sanitizing device according to the invention, wherein a cover member of the sanitizing device is in a closing position;
Fig. 2 shows the sanitizing device of Fig. 1, wherein the cover member is in an opening position;
Fig. 3 shows an electric shaving device of an embodiment of a shaving system according to the invention;
Fig. 4 shows a side view of a shaving unit of the shaving device of Fig. 3;
Fig. 5 shows a top view of the sanitizing device of Fig. 2 with the shaving unit of Fig. 4 being placed in a base member of the sanitizing device;
Fig. 6 shows a cross section of the sanitizing device of Fig. 1, with the shaving unit of Fig. 4 being placed in the sanitizing device; and
Fig. 7 shows an isometric cross section view of a base member of the sanitizing device of Fig. 1.

### DETAILED DESCRIPTION OF THE EMBODIMENTS

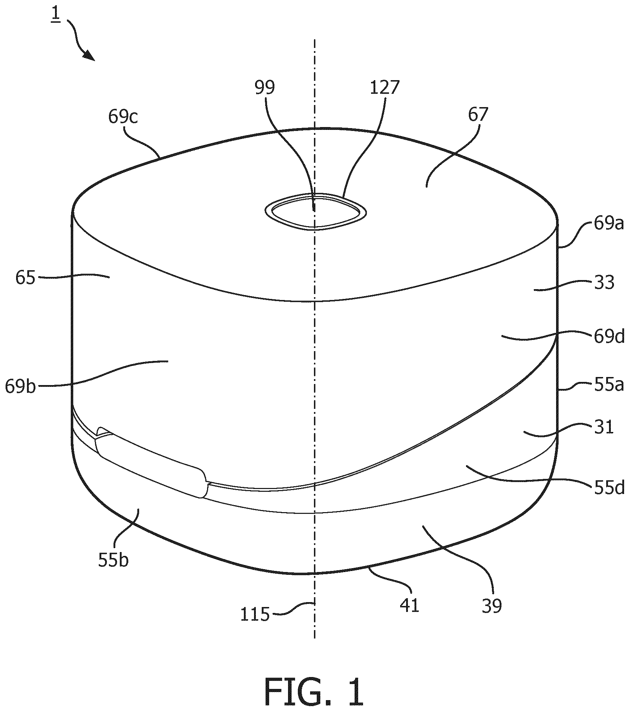

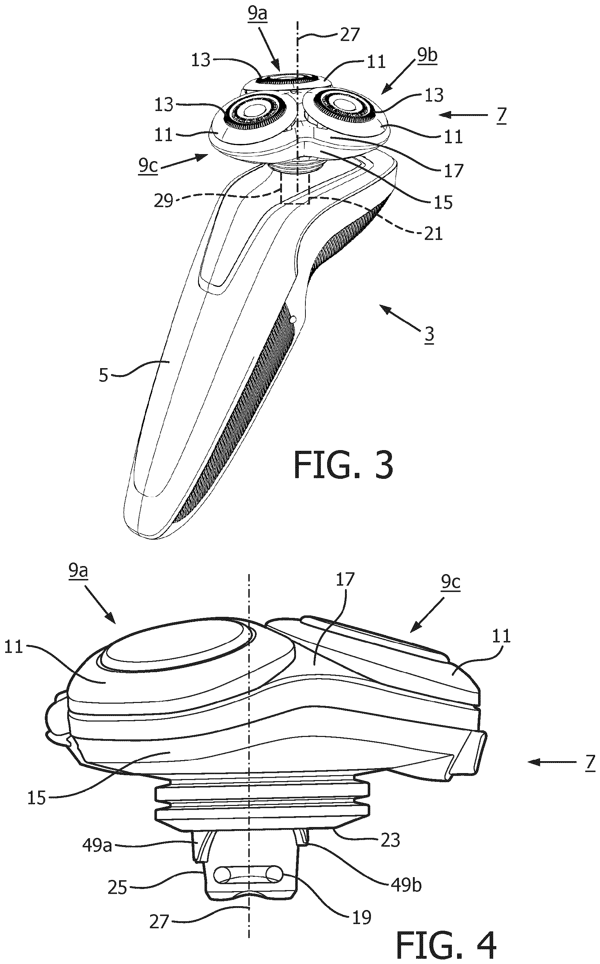

Fig. 1 shows an embodiment of a sanitizing device 1 according to the invention. The sanitizing device 1 is part of an embodiment of a shaving system according to the invention, wherein the shaving system comprises the sanitizing device 1 and an electric shaving device 3 as shown in Fig. 3. In this embodiment of the shaving system, the shaving device 3 comprises a main body 5 and a shaving unit 7, which is shown in detail in Fig. 4. The shaving device 3 is of a rotary type, wherein the shaving unit 7 comprises three hair-cutting units 9a, 9b, 9c. Each hair-cutting unit 9a, 9b, 9c has an external cutting member 11 having an annular shaving track 13 with hair-entry openings, and an internal cutting member (not visible in the figures) having an annular array of hair-cutting elements, wherein the internal cutting member is covered by and rotatable relative to the external cutting member 11. Such shaving devices with hair-cutting units of a rotary type are well known to the skilled person and, therefore, will not be described in further detail.

As shown in Figs. 3 and 4, the three hair-cutting units 9a, 9b, 9c of the shaving unit 7 are supported by a supporting member 15 of the shaving unit 7 and are arranged triangularly at a second main side 17 of the supporting member 15. The supporting member 15 is provided with a second coupling member 19 which is configured to be releasably couplable to a first coupling member 21 which is provided on the main body 5 of the shaving device 3 and is indicated only schematically in Fig. 3. The first coupling member 21 and the second coupling member 19 are configured for coupling and decoupling of the shaving unit 7 to and from the main body 5. For this purpose, the second coupling member 19 is arranged at a first main side 23 of the supporting member 15, which is opposite to the second main side 17 of the supporting member 15 and faces the main body 5 when the shaving unit 7 is coupled to the main body 5.

In the example shown, the first coupling member 21 and the second coupling member 19 are of a type as disclosed by EP2086729B2 in the name of the applicant. As shown in Fig. 4, the second coupling member 19 of the shaving unit 7 comprises a shaft-like coupling element 25 which protrudes from the first main side 23 of the supporting member 15 of the shaving unit 7. A central axis 27 of the shaft-like coupling element 25 extends centrally between the three triangularly arranged hair-cutting units 9a, 9b, 9c. The shaft-like coupling element 25 accommodates a single drive shaft (not visible in the figures) which will be coupled to a motor shaft of a motor (not visible in the figures) arranged in the main body 5 of the shaving device 3 when the shaving unit 7 is coupled to the main body 5. Thus, when the shaving unit 7 is coupled to the main body 5, said motor may drive the internal cutting members of the hair-cutting units 9a, 9b, 9c into rotation via said motor shaft, said single drive shaft arranged in the shaft-like coupling element 25, and a gear system (not visible in the figures) arranged in the supporting member 15 of the shaving unit 7. The shaft-like coupling element 25 is configured to be received by a coupling recess 29, which is provided in the main body 5 of the shaving device 3 (as schematically shown in Fig. 3) and accommodates the first coupling member 21. Further details of this releasable coupling structure between the main body 5 and the shaving unit 7 of the shaving device 3 are described in EP2086729B2.

It is to be noted that the invention is not limited to sanitizing devices for electric shaving devices of a type as described herein before with reference to Figs. 3 and 4, and that the invention is not limited to shaving systems that comprise a sanitizing device according to the invention and an electric shaving device of a type as described herein before with reference to Figs. 3 and 4. In particular, a sanitizing device according to the invention may be used for any type of electric shaving device comprising a main body and a shaving unit which is releasably couplable to the main body, wherein a first main side of the supporting member of the shaving unit is provided with a coupling member for releasable coupling of the shaving unit to the main body, and wherein at least one hair-cutting unit is arranged at a second main side of the supporting member opposite to the first main side. The one or more hair-cutting units may be of any known type, and hair-cutting units of different types may be provided on the shaving unit. The hair-cutting units may, for example, be of a linear reciprocating type, comprising an elongated and curved foil-type external cutting member with hair-entry openings and an elongated internal cutting member with a plurality of linearly arranged cutting blades which is arranged to linearly reciprocate relative to the external cutting member.

The sanitizing device 1 is configured to disinfect the shaving unit 7 of the shaving device 3 by means of disinfecting light. The disinfecting light may be ultraviolet (UV) light, in particular UV-C light, or light having a different wavelength having a disinfecting effect, such as blue light. Disinfection involves the inactivation of microorganisms by damaging their DNA and RNA, rendering them unable to reproduce and subsequently leading to their death. In particular, UV-C light is effective in inactivating, e.g., Escherichia Coli, Staphylococcus Aureus, Propionibacterium Acnes, and Pseudomonas Aeruginosa bacteria which may be present on the surfaces of the shaving unit 7 and the hair-cutting units 9a, 9b, 9c thereof after one or more shaving sessions using the shaving device 3. Inactivating these microorganisms prevents them from causing infections and inflammations of the skin tissue during a next shaving session using the shaving device 3.

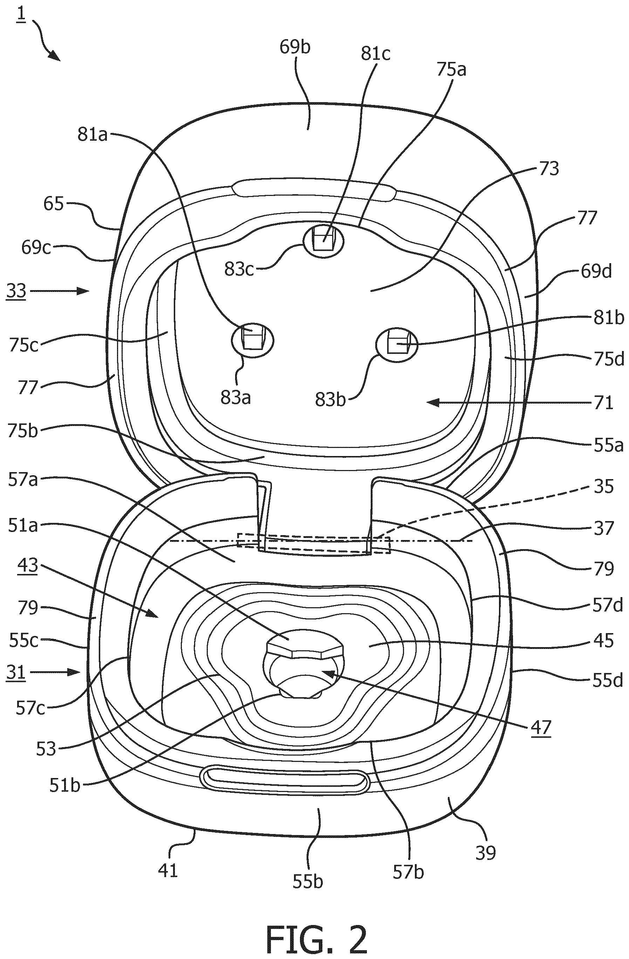

As shown in Figs. 1 and 2, the sanitizing device 1 comprises a base member 31 and a cover member 33. In the embodiment shown in Figs. 1 and 2, the cover member 33 is connected to the base member 31 by means of a hinge structure 35, which is indicated only schematically in Fig. 2 and which may be of any suitable type well known to the skilled person in the technical field of, e.g., openable and closable small box-like or case-like containers. By means of the hinge structure 35, the cover member 33 is pivotable relative to the base member 31 about a hinge axis 37 defined by the hinge structure 35. Fig. 1 shows the cover member 33 in a closing position relative to the base member 31, and Fig. 2 shows the cover member 33 in an opening position relative to the base member 31. The cover member 33 is pivotable relative to the base member 31 from the opening position to the closing position and vice versa. However, as already discussed hereinbefore, the invention is not limited to embodiments wherein the cover member is hinged relative to the base member, and the cover member may be moveable relative to the base member between a closing position and an opening position in alternative ways and by means of alternative structures. In the opening position, the cover member may, e.g., be completely separated from the base member.

As shown in Figs. 1 and 2, the base member 31 comprises a base member housing 39 having a bottom surface 41 configured for stable placement of the sanitizing device 1 on a horizontal external supporting surface, e.g., of a table. As shown in Fig. 2, a first cavity 43 is provided in the base member housing 39. In the opening position of the cover member 33, the first cavity 43 of the base member 31 is accessible for the user for placement of the shaving unit 7, i.e., when the shaving unit 7 has been decoupled from the main body 5 of the shaving device 3. The first cavity 43 is configured to at least partially receive the supporting member 15 of the shaving unit 7. The first cavity 43 has a first main inner surface 45 which is configured to support the first main side 23 of the supporting member 15 of the shaving unit 7 and, thereby, to define a predefined sanitizing position of the shaving unit 7 with respect to the base member 31. To enable the first main inner surface 45 of the first cavity 43 to support the first main side 23 of the supporting member 15 of the shaving unit 7 in a stable way, despite the presence of the shaft-like coupling element 25 protruding from the first main side 23 of the supporting member 15, the first cavity 43 further comprises a recess 47, which is provided at the first main inner surface 45 of the first cavity 43 and is configured to receive the shaft-like coupling element 25 when the shaving unit 7 is in the sanitizing position. As shown in Fig. 2, in this embodiment the recess 47 is arranged centrally at the first main inner surface 45 of the first cavity 43, i.e., in a position corresponding to the central position of the shaft-like coupling element 25 on the first main side 23 of the supporting member 15.

The recess 47 of the first cavity 43 enables a stable support of the shaving unit 7 by the first main inner surface 45 of the first cavity 43, and also limits the dimensions of the first cavity 43 required to accommodate the supporting member 15 of the shaving unit 7 despite the presence of the shaft-like coupling element 25 on the shaving unit 7. In addition, the recess 47 may assist the user placing the shaving unit 7 in the predefined sanitizing position within the base member 31 of the sanitizing device 1. For this purpose, as shown in Fig. 4, the shaft-like coupling element 25 comprises two diametrically oppositely arranged cam elements 49a, 49b having mutually different dimensions. In the sanitizing position of the shaving unit 7, said cam elements 49a, 49b closely fit into two correspondingly shaped notches 51a, 51b, which are provided at the recess 47 as shown in Fig. 2. Thereby, the user can place the shaft-like coupling element 25 only in a single predefined orientation within the recess 47, corresponding with the predefined sanitizing position of the shaving unit 7. To further assist the user placing the shaving unit 7 in the predefined sanitizing position within the base member 31 of the sanitizing device 1, the first main inner surface 45 of the first cavity 43 is also provided with a visual indication 53 of the circumferential contour of the shaving unit 7, i.e., in an orientation corresponding to the predefined sanitizing position of the shaving unit 7 in the base member 31. In the example shown, the visual indication 53 comprises a texture provided in the first main inner surface 45.

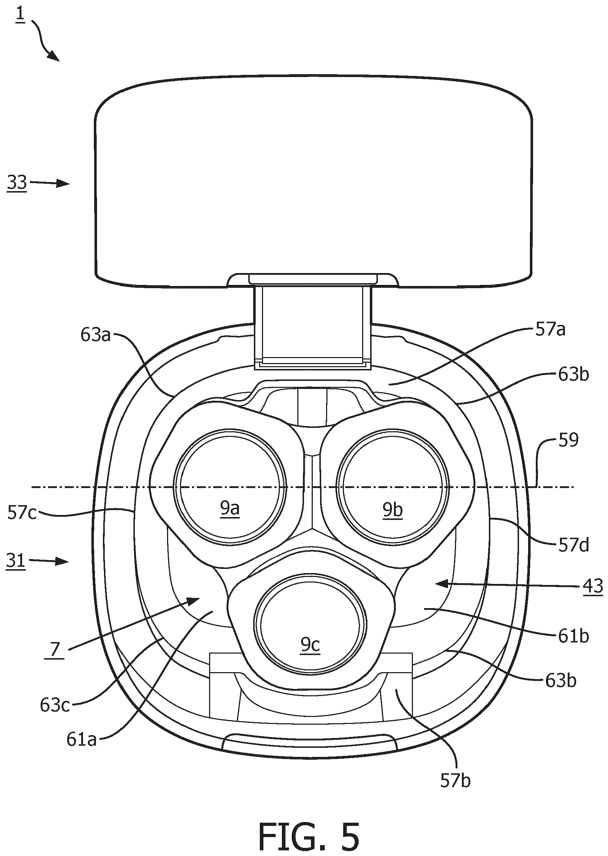

As shown in Figs. 1 and 2, the base member housing 39 of the base member 31 of the sanitizing device 1 has a generally square or quadrangular shape formed by mutually opposite first and second side walls 55a, 55b and mutually opposite third and fourth side walls 55c, 55d of the base member housing 39. As shown in Fig. 2, the first cavity 43 of the base member 31 also has a generally square or quadrangular shape, wherein the first main inner surface 45 of the first cavity 43 is surrounded by mutually opposite first and second side surfaces 57a, 57b of the first cavity 43 and by mutually opposite third and fourth side surfaces 57c, 57d of the first cavity 43 which each extend between the first and second side surfaces 57a, 57b. Fig. 5 shows a top view of the sanitizing device 1, with the cover member 33 in the opening position and with the shaving unit 7 being placed in the predefined sanitizing position within the first cavity 43 of the base member 31. As shown in Fig. 5, in the predefined sanitizing position of the shaving unit 7, a first one 9a and a second one 9b of the three hair-cutting units 9a, 9b, 9c of the shaving unit 7 are arranged adjacent to the first side surface 57a of the first cavity 43 on an imaginary line 59 which extends between the third and fourth side surfaces 57c, 57d of the first cavity 43. The third one 9c of the three hair-cutting units 9a, 9b, 9c of the shaving unit 7 is arranged in a central position relative to and adjacent to the second side surface 57b of the first cavity 43. As a result of this orientation of the shaving unit 7 within the generally square first cavity 43, two open spaces 61a, 61b are available, respectively, between the third side surface 57c of the first cavity 43 and the third one 9c of the three hair-cutting units 9a, 9b, 9c and between the fourth side surface 57d of the first cavity 43 and the third one 9c of the three hair-cutting units 9a, 9b , 9c when the shaving unit 7 is in the sanitizing position within the first cavity 43. Said two open spaces 61a, 61b are accessible for the user's fingers when placing the shaving unit 7 into the first cavity 43 and when removing the shaving unit 7 from the first cavity 43, thus facilitating the placement and removal of the shaving unit 7 by the user into and from the sanitizing device 1. As shown in Fig. 5, apart from these two open spaces 61a, 61b, the first, second, third and fourth side surfaces 57a, 57b, 57c, 57d of the first cavity 43 closely surround the shaving unit 7 in the sanitizing position, so that the dimensions of the first cavity 43 are minimized. As is further shown in Fig. 5, the first and second side surfaces 57a, 57b of the first cavity 43 are each connected to the third and fourth side surfaces 57c, 57d of the first cavity 43 by a respective one of four curved side-surface portions 63a, 63b, 63c, 63d of the first cavity 43. In particular, to enable a compact structure of the sanitizing device 1, the curved side-surface portions 63a, 63b, which connect the first side surface 57a with, respectively, the third side surface 57c and the fourth side surface 57d, are shaped to closely surround, respectively, the first one 9a and the second one 9b of the three hair-cutting units 9a, 9b, 9c of the shaving unit 7 in the sanitizing position of the shaving unit 7.

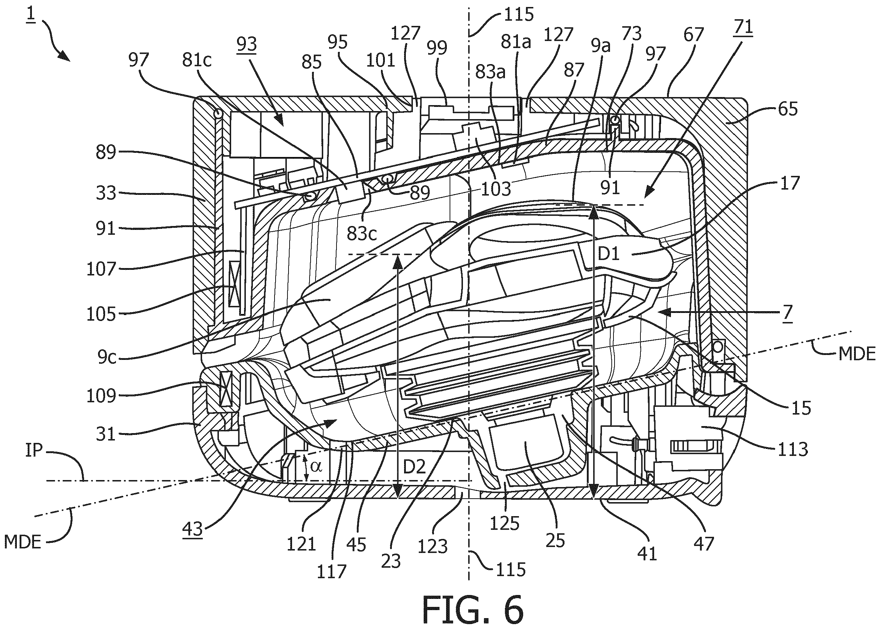

As shown in Figs. 1 and 2, the cover member 33 of the sanitizing device 1 comprises a cover member housing 65 having a top surface 67. The cover member housing 65 has a generally square or quadrangular shape which is similar to the shape of the base member housing 39 and is formed by mutually opposite first and second side walls 69a, 69b of the cover member housing 65 and mutually opposite third and fourth side walls 69c, 69d of the cover member housing 65. As shown in Fig. 2, the cover member 33 further comprises a second cavity 71 having a second main inner surface 73 which is surrounded by mutually opposite first and second side surfaces 75a, 75b of the second cavity 71 and by mutually opposite third and fourth side surfaces 75c, 75d of the second cavity 71 which each extend between the first and second side surfaces 75a, 75b. Thereby, the second cavity 71 of the cover member 33 also has a generally square or quadrangular shape which is similar to the shape of the first cavity 43 of the base member 31. In particular, a shape and dimensions of a circumferential edge portion 77 of the second cavity 71 of the cover member 33 are similar to a shape and dimensions of a circumferential edge portion 79 of the first cavity 43 of the base member 31 such that, in the closing position of the cover member 33, the circumferential edge portions 77, 79 of the first cavity 43 and the second cavity 71 are in mutual contact over the full circumferences of the base member 31 and the cover member 33. As a result, the first cavity 43 and the second cavity 71 are configured to fully enclose, in the closing position of the cover member 33 and with the shaving unit 7 being placed in the sanitizing position relative to the base member 31, the shaving unit 7 when being decoupled from the main body 5 of the shaving device 3. This is shown in Fig. 6, which shows a cross section of the sanitizing device 1 with the closing member 33 in its closing position relative to the base member 31 and with the shaving unit 7 being placed in the predefined sanitizing position within the sanitizing device 1. Fig. 6 also shows the first main side 23 of the supporting member 15 of the shaving unit 7 being supported by the first main inner surface 45 of the first cavity 43 of the base member 31. Fig. 6 further shows that the second cavity 71 of the cover member 33 is configured to receive, when the cover member 33 is in the closing position and the shaving unit 7 is in the predefined sanitizing position, the hair-cutting units 9a, 9b, 9c of the shaving unit 7.

As shown in Fig. 2, the embodiment of the sanitizing device 1 comprises three light sources 81a, 81b, 81c which are configured to generate the disinfecting light. The light sources 81a, 81b, 81c are arranged in the cover member 33 in a triangular configuration to each irradiate, from a respective one of three triangularly arranged light emitting areas 83a, 83b, 83c at the second main inner surface 73 of the second cavity 71 of the cover member 33, a respective one of the three hair-cutting units 9a, 9b, 9c of the shaving unit 7 with the disinfecting light when the cover member 33 is in the closing position relative to the base member 31 and the shaving unit 7 is in the predefined sanitizing position. It is noted that, with respect to the present invention, the term "light emitting area" means an area at the second main inner surface of the second cavity of the cover member from which the disinfecting light, which is generated by a light source associated with said light emitting area, is emitted. In some embodiments, such as in the embodiment of the sanitizing device 1 as shown in Fig. 2, the light source may be arranged at said light emitting area. In other embodiments, the light source may be arranged in the cover member at a distance from the second main inner surface of the second cavity, and the disinfecting light generated by the light source may be transmitted to said light emitting area via, e.g., a light guide. In such alternative embodiments, the light emitting area may be a light exit window of an optically transparent material provided at the second main inner surface of the second cavity, or an end surface of an optical light guide via which the disinfecting light is transmitted from the light source.

In the embodiment shown in Fig. 2, the three light sources 81a, 81b, 81c and the three light emitting areas 83a, 83b, 83c associated with the light sources 81a, 81b, 81c are thus arranged in positions at the second main inner surface 73 of the second cavity 71 corresponding to the positions of the three hair-cutting units 9a, 9b, 9c at the second main side 17 of the supporting member 15 of the shaving unit 7, i.e., in the sanitizing position of the shaving unit 7 within the sanitizing device 1. As shown in Fig. 6 for the hair-cutting unit 9c and the light source 81c and the light emitting area 83c, in this embodiment the light sources 81a, 81b, 81c and associated light emitting areas 83a, 83b, 83c are arranged in positions relatively close to the associated hair-cutting unit 9a, 9b, 9c in the sanitizing position of the shaving unit 7 with the cover member 33 in its closing position relative to the base member 31. This results both in an effective disinfection of the hair-cutting units 9a, 9b, 9c by the disinfecting light emitted from the light emitting areas 83a, 83b, 83c and in relatively small dimensions of the sanitizing device 1. It is noted that the invention also covers embodiments wherein the sanitizing device has a different number of light sources and/or light emitting areas provided at the second main inner surface of the second cavity of the cover member. In the embodiment of the shaving system with the shaving unit 7 having the three hair-cutting units 9a, 9b, 9c, e.g., a single light source and/or a single light emitting area may be centrally arranged at the second main inner surface of the second cavity to irradiate all three hair-cutting units 9a, 9b, 9c of the shaving unit 7. Alternatively, more than one light source and/or more than one light emitting area may be provided per individual hair-cutting unit of the shaving unit, also depending on the shape and dimensions of the hair-cutting unit(s) of the shaving unit.

As shown in Fig. 6, by supporting the shaving unit 7 at the first main side 23 of the supporting member 15 by means of the first main inner surface 45 of the first cavity 43, and by providing the light emitting areas 83a, 83b, 83c at the second main inner surface 73 of the second cavity 71 to enable the irradiation of the hair-cutting units 9a, 9b, 9c arranged at the second main side 17 of the supporting member 15, the shape and dimensions of the sanitizing device 1 may be fit close to the shape and dimensions of the shaving unit 7, so that the dimensions of the sanitizing device 1 can be minimized to achieve a compact and light structure of the sanitizing device 1. Because in the closing position of the cover member 33 the first cavity 43 and the second cavity 71 are fully closed, the disinfecting light irradiated from the light emitting areas 83a, 83b, 83c at the second main inner surface 73 of the second cavity 71 is prevented from escaping from the first cavity 43 and the second cavity 71 towards the environment of the sanitizing device 1 and, thereby, prevented from injuring the user's eyes.

In the embodiment of the sanitizing device 1, the three light sources 81a, 81b, 81c each comprise a UV-C LED. As shown in Fig. 6, the light sources 81a, 81b, 81c are arranged on a printed circuit board (PCB) 85 which is arranged in the cover member 33 between the second main inner surface 73 of the second cavity 71 and the top surface 67 of the cover member housing 65. The PCB 85 is mounted close to an inner wall 87 of the second cavity 71 which carries the second main inner surface 73. The light sources 81a, 81b, 81c are each arranged in a respective opening provided in said inner wall 87, said respective opening thus corresponding to the respective light emitting area 83a, 83b, 83c provided at the second main inner surface 73 for the respective light source 81a, 81b, 81c. In Fig. 6, only the opening for the light source 81c is visible. As further shown in Fig. 6 for the opening for the light source 81c, a sealing ring 89 is arranged between said inner wall 87 and the PCB 85 around the opening for each light source 81a, 81b, 81c to prevent the penetration of moisture from the second cavity 71 to the electronic components of the PCB 85 via said openings for the light sources 81a, 81b, 81c. As further shown in Fig. 6, the inner wall 87 of the second cavity 71 is integrally provided with an upright cylindrical sealing wall 91 on its upper surface. The cylindrical sealing wall 91 defines a receiving space 93 for the PCB 85 together with the inner wall 87 and an upper wall 95 of the cover member housing 65 which carries the top surface 67. A sealing ring 97 is provided between an upper edge of the cylindrical sealing wall 91 and the upper wall 95 of the cover member housing 65 to prevent the penetration of moisture into the receiving space 93 for the PCB 85.

As shown in Fig. 1, the sanitizing device 1 further comprises a user-operable on/off button 99, which is arranged at the top surface 67 of the cover member 33. As shown in Fig. 6, the on/off button 99 is arranged in an opening 101 provided in the upper wall 95 of the cover member housing 65 and interacts with an electrical switch 103, which is mounted on the PCB 85 and is electrically connected to the PCB 85.

As further shown in Fig. 6, the sanitizing device 1 has a detector 105 which is provided in the cover member 33 on an extension part 107 of the PCB 85. The detector 105 is configured and arranged to detect whether the cover member 33 is in the closing position and to disable the light sources 81a, 81b, 81c from generating the disinfecting light when the cover member 33 is in the opening position relative to the base member 31. The detector 105 thus prevents the activation of the light sources 81a, 81b, 81c when the cover member 33 is in the opening position and, thereby, prevents injury to the user's eyes by the disinfecting light when the cover member 33 is in the opening position. In the embodiment of the sanitizing device 1 as shown in Fig. 6, the detector 105 comprises a magnetic reed sensor switch, which is well known to the skilled person. The magnetic reed sensor switch cooperates with a permanent magnet 109, which is arranged in the base member 31. In the closing position of the cover member 33, the magnetic reed sensor switch is closed by the magnetic field of the permanent magnet 109. This will enable the activation of the light sources 81a, 81b, 81c by means of the on/off button 99 and the electrical switch 103. When the cover member 33 is pivoted out of the closing position, the magnetic reed sensor switch will be opened and the power supply to the light sources 81a, 81b, 81c will be disabled. The magnetic reed sensor switch will already be opened when the cover member 33 is pivoted over a relatively small angle from the closing position. Instead of the magnetic reed sensor switch, any other type of suitable detector 105 may be used, such as a Hall sensor or a mechanical switch sensor.

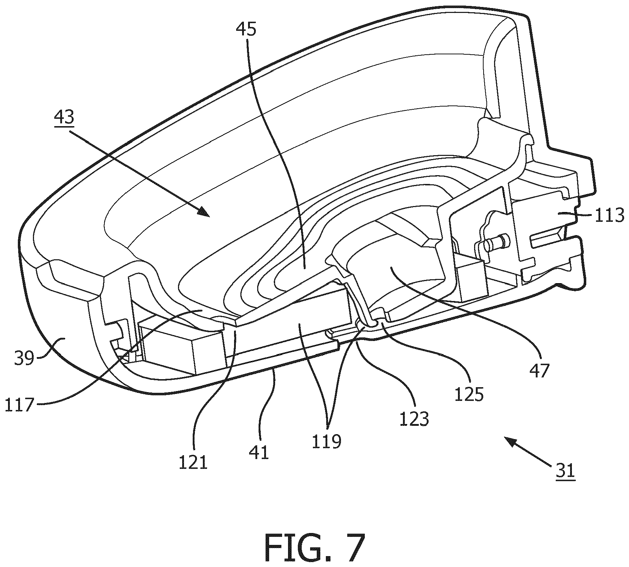

All main electrical components of the sanitizing device 1, such as the light sources 81a, 81b, 81c, the PCB 85, the electrical switch 103, the detector 105 and an electrical power supply (not shown in figures) for the light sources 81a, 81b, 81c, may thus be arranged in the cover member 33, in particular within the sealed receiving space 93 provided in the cover member 33. This further simplifies the structure of the sanitizing device 1. As shown in Fig. 7, which shows an isometric cross section view of the base member 31 of the sanitizing device 1, a power socket 113 for receiving an electrical power cable may be arranged in the base member 31. The power socket 113 may be connected to the PCB 85 in the cover member 35 via electrical wires (not shown in the figures). Preferably, the electrical wires may extend through the hinge structure 35, or very close to the hinge structure 35, in order not to hinder the pivoting motion of the cover member 33 relative to the base member 31.

As shown in Figs. 1 and 6, the base member 31 of the sanitizing device 1 has a central axis 115 which extends perpendicularly to the bottom surface 41 of the base member housing 39. As shown in Fig. 6, which shows a cross section of the sanitizing device 1 including the central axis 115, the first main inner surface 45 of the first cavity 43 has a main direction of extension MDE in said cross section which is inclined relative to an imaginary plane IP extending perpendicularly to the central axis 115. Fig. 6 shows an inclination angle α enclosed by the main direction of extension MDE of the first main inner surface 45 and the imaginary plane IP. As a result of the inclination angle α, the first main inner surface 45 of the first cavity 43 is configured such that, in the predefined sanitizing position of the shaving unit 7 as shown in Fig. 5, the first one 11a and the second one 11b of the three hair-cutting units 11a, 11b, 11c of the shaving unit 7 are arranged at a first distance D₁ from the bottom surface 41 of the base member housing 39, and the third one 11c of the three hair-cutting units 11a, 11b, 11c is arranged at a second distance D₂ from the bottom surface 41 which is smaller than said first distance D₁. Said first and second distances D₁ and D₂ are indicated in Fig. 6.

When the bottom surface 41 of the base member 31 is placed on a horizontal external supporting surface, e.g., of a table, the first main inner surface 45 of the first cavity 43 and the shaving unit 7 supported by the first main inner surface 45 are both inclined relative to the horizontal imaginary plane IP, as shown in Fig. 6. The inclination of the first main inner surface 45 of the first cavity 43 improves the visibility of the first cavity 43 for the user when placing the shaving unit 7 in the base member 31. Furthermore, the inclinations of the first main inner surface 45 of the first cavity 43 and the shaving unit 7 enable any water or cleaning fluid, that may be present on the shaving unit 7 when placing the shaving unit 7 in the base member 31, to drip from the shaving unit 7 onto the first main inner surface 45 and to flow towards a single lowest area 117 of the first cavity 43 indicated in Figs. 6 and 7. Said water or cleaning fluid may be present on the shaving unit 7 because, for an effective disinfecting process by means of the disinfecting light, the user of the sanitizing device 1 may be recommended to first clean the shaving unit 7, and in particular the hair-cutting units 9a, 9b, 9c thereof, by means of water or a cleaning fluid in order to remove any debris from the shaving unit 7 and the hair-cutting units 9a, 9b, 9c before using the sanitizing device 1.

To enable any water or cleaning fluid to be drained from the first cavity 43, the base member 31 of the sanitizing device 1 has a drainage conduit 119, as shown in Fig. 7, which connects a first drainage opening 121 with a second drainage opening 123. The first drainage opening 121 is provided in the first main inner surface 45 of the first cavity 43 in a position close to or at the lowest area 117 of the first cavity 43. The second drainage opening 123 is provided in the bottom surface 41 of the base member 31. As shown in Fig. 6, the first drainage opening 121 is arranged in a position which is adjacent to the third one 9c of the three hair-cutting units 9a, 9b, 9c of the shaving unit 7 when the shaving unit 7 is in the sanitizing position. As shown in Fig. 7, the drainage conduit 119 of the base member 31 also connects a third drainage opening 125, which is provided in the recess 47 of the first cavity 43, with the second drainage opening 123 in the bottom surface 41 of the base member 31. By providing the drainage conduit 119, the user does not need to dry or fully dry the shaving unit 7 before placing the shaving unit 7 in the sanitizing device 1. Water or cleaning fluid present on the shaving unit 7 when placing the shaving unit 7 in the sanitizing device 1 may drip from the shaving unit 7 and flow to the lowest area 117 of the first cavity 43 or into the recess 47 of the first cavity 43. From there, the water or cleaning fluid may be drained via, respectively, the first drainage opening 121 and the third drainage opening 125 and via the drainage conduit 119 and the second drainage opening 123 out of the base member 31. Allowing the water or cleaning fluid to be drained from the sanitizing device 1 mitigates the risk of an ineffective disinfecting process by the disinfecting light as a result of the presence of water or cleaning fluid on the shaving unit 7. As shown in Fig. 7, the first drainage opening 121 and the third drainage opening 125 are not arranged in line relative to the second drainage opening 123 to reduce as much as possible the transmission of any disinfecting light via the drainage conduit 119 to the second drainage opening 123.

In order to disinfect the shaving unit 7 of the shaving device 3 by means of the sanitizing device 1, the user needs to decouple the shaving unit 7 from the main body 3 and, preferably, first clean the shaving unit 7 by means of water or a cleaning fluid. Then the user needs to open the sanitizing device 1, by pivoting the cover member 33 into the opening position, and place the shaving unit 7 in the sanitizing position into the first cavity 43 of the base member 31, as indicated by the visual indication 53 which is provided at the first main inner surface 45 of the first cavity 43. After closing the sanitizing device 1, by pivoting the cover member 33 into the closing position, the user needs to press the on/off button 99. This will activate the light sources 81a, 81b, 81c to generate the disinfecting light for a predefined time period, e.g., 10 minutes. The active state of the light sources 81a, 81b, 81c is indicated by means of an annular light indicator 127 which is arranged around the on/off button 99, as shown in Fig. 1, and is mounted on the PCB 85 in a position surrounding the electrical switch 103, as schematically shown in Fig. 6. The light indicator 127 may, e.g., generate pulsing light during the active state of the light sources 81a, 81b, 81c, and will be switched off after the predefined time period to indicate that the sanitizing process is completed.

Variations to the disclosed embodiments can be understood and effected by those skilled in the art in practicing the principles and techniques described herein, from a study of the figures, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference numbers in the claims should not be construed as limiting the scope of the claims.

## Claims

1. A sanitizing device (1) configured to disinfect a shaving unit (7) of an electric shaving device (3) by means of disinfecting light, wherein the shaving device comprises a main body (5) provided with a first coupling member (21) and the shaving unit comprises:
- a supporting member (15) provided with a second coupling member (19) configured to be releasably couplable to the first coupling member for coupling and decoupling of the shaving unit to and from the main body, wherein the second coupling member is arranged at a first main side (23) of the supporting member which faces the main body when the shaving unit is coupled to the main body; and
- at least one hair-cutting unit (a, 9b, 9c) arranged at a second main side (17) of the supporting member opposite to the first main side;
wherein the sanitizing device (1) comprises:
- a base member (31) comprising a first cavity (43) configured to at least partially receive the supporting member (15) of the shaving unit (7) in a condition of the shaving unit decoupled from the main body (5), said first cavity having a first main inner surface (45) configured to support the first main side (23) of the supporting member in a predefined sanitizing position of the shaving unit with respect to the base member;
- a cover member (33) having a closing position and an opening position relative to the base member, and further having a second cavity (71) configured to receive, when the cover member is in the closing position and the shaving unit is in the predefined sanitizing position, the at least one hair-cutting unit (9a, 9b, 9c) of the shaving unit, said second cavity having a second main inner surface (73);
and
- at least one light source (81a, 81b, 81c) configured to generate the disinfecting light and arranged in the cover member to irradiate, from a light emitting area (83a, 83b, 83c) at the second main inner surface, the at least one hair-cutting unit with the disinfecting light when the cover member is in the closing position and the shaving unit is in the predefined sanitizing position;
wherein the first cavity and the second cavity are configured to fully enclose the shaving unit in its condition decoupled from the main body in the closing position of the cover member when the shaving unit is in the sanitizing position.

2. The sanitizing device (1) as claimed in claim 1, wherein the second coupling member (19) of the shaving unit (7) comprises a shaft-like coupling element (25) which protrudes from the first main side (23) of the supporting member (15) of the shaving unit, and wherein the first cavity (43) comprises a recess (47) which is provided at the first main inner surface (45) of the first cavity and configured to receive the shaft-like coupling element when the shaving unit is in the sanitizing position.

3. The sanitizing device (1) as claimed in claim 2, wherein the shaving unit (7) comprises three rotary-type hair-cutting units (9a, 9b, 9c) arranged triangularly at the second main side (17) of the supporting member (15), and wherein a central axis (27) of the shaft-like coupling element (25) extends centrally between the three hair-cutting units, and wherein the recess (47) of the first cavity (43) is arranged centrally at the first main inner surface (45) of the first cavity.

4. The sanitizing device (1) as claimed in claim 2 or 3, wherein the base member (31) has a drainage conduit (119) configured to connect a first drainage opening (125), which is provided in the recess (47) of the first cavity (43), with a second drainage opening (123) which is provided in a bottom surface (41) of the base member.

5. The sanitizing device (1) as claimed in claim 1, wherein the base member (31) has a bottom surface (41) and a central axis (115) extending perpendicularly to the bottom surface, and wherein the first main inner surface (45) of the first cavity (43), seen in a cross section which includes the central axis, has a main direction of extension (MDE) which is inclined relative to an imaginary plane (IP) extending perpendicularly to the central axis of the base member.

6. The sanitizing device (1) as claimed in claim 5, wherein the shaving unit (7) comprises three rotary-type hair-cutting units (9a, 9b, 9c) arranged triangularly at the second main side (17) of the supporting member (15), and wherein the first main inner surface (45) of the first cavity (43) is configured such that, in the predefined sanitizing position of the shaving unit, a first one (9a) and a second one (9b) of the three hair-cutting units of the shaving unit are arranged at a first distance (D1) from the bottom surface (41) of the base member (31), and a third one (9c) of the three hair-cutting units of the shaving unit is arranged at a second distance (D2) from the bottom surface which is smaller than the first distance.

7. The sanitizing device (1) as claimed in claim 6, wherein the base member (31) has a drainage conduit (119) configured to connect a first drainage opening (121), which is provided in the first main inner surface (45) of the first cavity (43), with a second drainage opening (123) which is provided in a bottom surface (41) of the base member, wherein the first drainage opening is arranged in a position which is adjacent to the third one (9c) of the three hair-cutting units (9a, 9b, 9c) of the shaving unit (7) when the shaving unit is in the sanitizing position.

8. The sanitizing device (1) as claimed in claim 1, wherein the shaving unit (7) comprises three rotary-type hair-cutting units (9a, 9b, 9c) arranged triangularly at the second main side (17) of the supporting member (15), and wherein the first main inner surface (45) of the first cavity (43) is:
- surrounded by mutually opposite first and second side surfaces (57a, 57b) of the first cavity and by mutually opposite third and fourth side surfaces (57c, 57d) of the first cavity which each extend between the first and second side surfaces;
- configured such that, in the predefined sanitizing position of the shaving unit, a first one (9a) and a second one (9b) of the three hair-cutting units of the shaving unit are arranged adjacent to the first side surface (57a) on an imaginary line (59) extending between the third and fourth side surfaces (57c, 57d), and a third one (9c) of the three hair-cutting units of the shaving unit is arranged in a central position relative to and adjacent to the second side surface (57b).

9. The sanitizing device (1) as claimed in claim 8, wherein the first and second side surfaces (57a, 57b) of the first cavity (43) are each connected to the third and fourth side surfaces (57c, 57d) of the first cavity by a respective one of four curved side-surface portions (63a, 63b, 63c, 63d) of the first cavity.

10. The sanitizing device (1) as claimed in any of the claims 1-9, wherein the cover member (33) is connected to the base member (31) by means of a hinge structure (35), and wherein the cover member is pivotable relative to the base member between the opening position and the closing position about a hinge axis (37) defined by the hinge structure.

11. The sanitizing device (1) as claimed in claim 10, wherein the cover member (33) comprises a detector (105), which is configured and arranged to detect whether the cover member is in the closing position and to disable the at least one light source (81a, 81b, 81c) from generating the disinfecting light when the cover member is in the opening position.

12. The sanitizing device (1) as claimed in claim 1, wherein the shaving unit (7) comprises three rotary-type hair-cutting units (9a, 9b, 9c) arranged triangularly at the second main side (17) of the supporting member (15), and wherein the sanitizing device comprises three light sources (81a, 81b, 81c) arranged triangularly in the cover member (33) to each irradiate, from a respective one of three triangularly arranged light emitting areas (83a, 83b, 83c) at the second main inner surface (73) of the second cavity (71), a respective one of the three hair-cutting units with the disinfecting light when the cover member is in the closing position and the shaving unit is in the predefined sanitizing position.

13. The sanitizing device (1) as claimed in claim 1, wherein the at least one light source (9a, 9b, 9c) is arranged on a printed circuit board (85) which is arranged in the cover member (33) between the second main inner surface (73) of the second cavity (71) and a top surface (67) of the cover member.

14. The sanitizing device (1) as claimed in claim 13, wherein the sanitizing device comprises a user-operable on/off button (99) arranged at the top surface (67) of the cover member (33) and electrically connected to the printed circuit board (85).

15. A shaving system comprising an electric shaving device (3) and a sanitizing device (1) as claimed in any of the claims 1-14 configured to disinfect a shaving unit (7) of the shaving device by means of disinfecting light, wherein the shaving device comprises a main body (5) provided with a first coupling member (21), and wherein the shaving unit of the shaving device comprises:
- a supporting member (15) provided with a second coupling member (19) configured to be releasably couplable to the first coupling member for coupling and decoupling of the shaving unit to and from the main body, wherein the second coupling member is arranged at a first main side (23) of the supporting member which faces the main body when the shaving unit is coupled to the main body; and
- at least one hair-cutting unit (9a, 9b, 9c) arranged at a second main side (17) of the supporting member opposite to the first main side.
